# EUROPEAN PATENT SPECIFICATION

(11) **EP 2 628 485 B1**
(45) Date of publication and mention of the grant of the patent: **01.06.2016**
(21) Application number: 10858279.2
(22) Date of filing: 14.10.2010
(51) Int. Cl.: A61K 36/185, A61P 5/30, A61P 19/10, A61P 35/04, A61P 9/00, A61K 36/10, A61K 31/122, A61K 31/353, A61K 31/7034, A61K 31/7048, A61K 36/18

(54) **NEW USE OF CHEMICAL INGREDIENTS IN CYNOMORIUM AS PHYTOESTROGEN**
NEUE VERWENDUNG VON CHEMISCHEN BESTANDTEILEN AUS CYNOMORIUM ALS PHYTOÖSTROGENE
NOUVEL USAGE D'INGRÉDIENTS CHIMIQUES DANS LE CYNOMORIUM COMME PHYTOESTROGÈNE

(43) Date of publication of application: 21.08.2013
(73) Proprietor: Tianjin University Of Traditional Chinese Medicine, Tianjin 300193 (CN)
(72) Inventor: GAO, Xiumei, Tianjin 300193 (CN)
(74) Representative: Santarelli
(86) International application number: PCT/CN2010/001611
(87) International publication number: WO 2012/048446

(56) References cited:
- US-A1- 2001 024 664
- US-A1- 2009 312 437
- DATABASE WPI Week 200444 Thomson Scientific, London, GB; AN 2004-465594 XP002719680, & KR 2004 0019513 A (UNIV KOREA HANKYONG NAT PRESIDENT) 6 March 2004 (2004-03-06)
- DATABASE WPI Week 200145 Thomson Scientific, London, GB; AN 2001-421066 XP002719681, & JP 2001 114675 A (FANKERU KK) 24 April 2001 (2001-04-24)
- MATSUDA H ET AL: "Phytoestrogens from the roots of Polygonum cuspidatum (Polygonaceae): Structure-requirement of hydroxyanthraquinones for estrogenic activity", BIOORGANIC & MEDICINAL CHEMISTRY LETTERS, PERGAMON, AMSTERDAM, NL, vol. 11, no. 14, 23 July 2001 (2001-07-23) , pages 1839-1842, XP002268646, ISSN: 0960-894X, DOI: 10.1016/S0960-894X(01)00318-3
- ROSENGREN R J: "Catechins and the treatment of breast cancer: Possible utility and mechanistic targets", IDRUGS, CURRENT DRUGS LTD, GB, vol. 6, no. 11, 1 November 2003 (2003-11-01), pages 1073-1078, XP009175976, ISSN: 1369-7056
- ZHANG C:Z. ET AL.: 'In vitro estrogenic activities of Chinese medicinal plants traditionally used for the management of menopausal symptoms' JOURNAL OF ETHNOPHARMACOLOGY vol. 98, 2005, pages 295 - 300, XP027757535

## Description

### TECHNICAL FIELD

The present invention relates to new use of several chemical ingredients from traditional Chinese medicine *Herba Cynomorii,* and particularly to new use of flavone compounds and anthraquinone compounds from *Herba Cynomorii* as phytoestrogen.

### BACKGROUND ART

*Herba Cynomorii* is the dry chylocaulous of *Cynomorium songaricum* Rupr. *Herba Cynomorii* has sweet flavor and warm nature, belongs to liver, kidney and large intestine meridians, and is an unique rare natural psammophyte. It has functions of reinforcing kidney Yang, benefiting blood and essence, loosening bowel and relieving constipation, and may be used for the treatment of impotence and seminal emission, soreness and weakness of waist and knees, dryness of intestine and constipation, blood exhaustion, hematuria, neurasthenia, etc. The studying of chemical ingredients of *Herba Cynomorii* indicates that *Herba Cynomorii* contains flavones, triterpenes, saccharides and glycosides, tannins, steroids, volatile ingredients, organic acids, amino acids, inorganic elements and ions, in which the flavones include (+)-catechin, (-)-catechin, naringenin 4'-O-glycopyranoside, and naringenin is glycoside of aglycon.

Estrogens are critical regulatory hormones for female, which are mainly produced by ovary and placenta, and estrogens in their chemical structure belong to steroid hormones, have liposolubility and many target tissues including reproductive system, bones, cardiovascular. Estrogens generally are divided into two groups: steroid estrogens and non-steroid estrogens. Steroid estrogens are endogenous ingredients secreted by ovary of mammals. So far, three steroid estrogens are found in human body: estradiol, estriol separated from urine of gravida, and estrone, in which estradiol has the strongest activity. Steroid estrogens have important functions in sustaining life, regulating sexual function, physical development, immune-regulation, treatment of skin diseases and procreation. Non-steroid estrogens mainly include stilbene glycoside compounds. It is generally believed that estrogens generate physiological effects by binding to corresponding estrogen receptors (ER) in cells, thereby inducing a series of downstream events such as inducing and starting transcription. The affinity of non-steroid estrogens to estrogen receptor is 1/100 or 1/1000 of that of estradiol. Since the long-term clinical use of estrogens may result in breast cancer, endometriosis and carcinogenicity, the alternates of estrogens are in request. Phytoestrogens (PE) are non-steroid compounds generally existing in plants, and may bind to *in vivo* ER, and epidemiologic studies and animal tests confirm that phytoestrogens have good prophylactic and therapeutical effects in the prevention of tumors and treatment of cardiovascular diseases and osteoporosis, improvement of menopausal symptom, may protect nervous system and show none of the above adverse effects. Hence, phytoestrogen are considered as alternates of natural estrogens. Although the affinity of PE to estrogen receptor is only 1/500-1/1000 of that of estrogens, PE has less side-effect in hormone replacement therapy (HRT), and draws huge attentions from researchers and is promising in HRT.

Estrogens have a wide scope of physical functions in growth, development, cell differentiation and reproductive system in organisms. Estrogens bring into various biological effects mainly through ER. There are two subtypes of ER, ERα and ERβ, which have different expression levels in many organs and cells in human body. The two subtypes have difference in expression level in the cells of same one tissue, and the same subtype has differences in expression level in the cells of difference tissues. Hence, the effects of interaction between any ligand and ERα and ERβ are complicated. ERα and ERβ have difference in tissue distribution, in which uterus, mammary gland, oviduct, vaginal epithelium mainly express ERα and have weak expression of ERβ, while ovary has predominant expression of ERβ, most existing in granular cells, and vascular smooth muscle cells mainly express ERβ.

Women in climacteric period have depleted ovary, reduced estrogen secretion, and thus various physiological processes mediated by estrogens are influenced, thereby bringing about physiological and psychological symptoms such as palpitation, oppression in chest, apprehension, depression, easy excitation, insomnia, hypomnesis, these symptoms are generally called as climacteric syndrome; the osteoporosis caused by the reduction of estrogen secretion is called degenerative osteoporosis. In order to treat and/or prevent the above disorders, one beneficial method is to replenish estrogens. However, it is found in many years of clinical practices that the risk of breast cancer attack increases greatly after long-term administration of estrogens. Hence, it is still desired by those skilled in the art to find phytoestrogens that have an activity of estrogens and/or no side-effects of estrogens, so as to provide a new therapeutical option for clinical application.

The estrogenic activity of 70% EtOH extracts of Cynomorium songaricum was examined in Zhang et al., "In vitro estrogenic activities of Chinese medicinal plants traditionnaly used for the management of menopausal symptoms*".*

### DESCRIPTION OF THE INVENTION

The object of the present invention is to provide phytoestrogens that have an activity of estrogens and/or no side-effects of estrogens, so as to provide a new therapeutical option for clinical application. The inventors of the present invention have surprisingly found that chemical ingredients, such as flavone and anthraquinone compounds, extracted from Chinese medicine *Herba Cynomorii,* have established activities of estrogens, and may be useful in the estrogen replacement therapy as phytoestrogens. The present invention has been accomplished on the basis of the above findings.

### Summary of the Invention

In a first aspect , this document features the use of *Herba Cynomorii* in the manufacture of a medicament for the treatment and/or prophylaxis of a disease associated with insufficient secretion of estrogens in a mammal (e.g., human).

The first aspect of the present invention provides a *Herba Cynomorii* extract, which comprises catechin, (-)epicatechin-3-*O*-gallate, naringenin-4'-*O*-glucoside, and phlorizin, and optionally chrysophanol and/or emodin,.

The second aspect of the present invention provides one or more active ingredients selected from the group consisting of: chrysophanol, emodin, catechin, (-)epicatechin-3-*O*-gallate, naringenin-4'-*O*-glucoside, and phlorizin, which are obtainable from *Herba Cynomorii.*

The third aspect of the present invention provides the use of a *Herba Cynomorii* extract in the manufacture of a medicament for the treatment and/or prophylaxis of a disease associated with insufficient secretion of estrogens in a mammal (e.g., human).

The fourth aspect of the present invention provides the use of catechin, (-)epicatechin-3-*O*-gallate, naringenin-4'-*O*-glucoside, and phlorizin, and optionallychrysophanol and/or emodin, in the manufacture of a medicament for the treatment and/or prophylaxis of a disease associated with insufficient secretion of estrogens in a mammal (e.g., human).

The fifth aspect of the present invention provides the use of *Herba Cynomorii* in the manufacture of a medicament as phytoestrogen.

The sixth aspect of the present invention provides use of *Herba Cynomorii* extract in the manufacture of a medicament as phytoestrogen.

The seventh aspect of the present invention provides use of catechin, (-)epicatechin-3-*O*-gallate, naringenin-4'-*O*-glucoside, and phlorizin, and optionally chrysophanol and/or emodin, in the manufacture of a medicament as phytoestrogen.

The eighth aspect of the present invention provides a pharmaceutical composition for the treatment and/or prophylaxis of a disease associated with insufficient secretion of estrogens in a mammal (e.g., human), which comprises a therapeutically and/or prophylactically effective amount of *Herba Cynomorii* extract and optionally a pharmaceutically acceptable excipient.

The ninth aspect of the present invention provides a pharmaceutical composition for the treatment and/or prophylaxis of a disease associated with insufficient secretion of estrogens in a mammal (e.g., human), which comprises a therapeutically and/or prophylactically effective amount of the pharmaceutically active ingredients catechin, (-)epicatechin-3-*O*-gallate, naringenin-4'-*O*-glucoside, and phlorizin, and optionally chrysophanol and/or emodin, and optionally a pharmaceutically acceptable excipient.

The tenth aspect of the present invention provides a method for the treatment and/or prophylaxis of a disease associated with insufficient secretion of estrogens in a subject in need thereof, which comprises administering to the subject a therapeutically and/or prophylactically effective amount of a *Herba Cynomorii* extract or the active ingredients catechin, (-)epicatechin-3-*O*-gallate, naringenin-4'-*O*-glucoside, and phlorizin, and optionally chrysophanol and/or emodin.

### Detailed Description of the Invention

In a first aspect , this document features the use of *Herba Cynomorii* in the manufacture of a medicament for the treatment and/or prophylaxis of a disease associated with insufficient secretion of estrogens in a mammal (e.g., human).

According to the use of any embodiment of the first aspect, the disease associated with insufficient secretion of estrogens is selected from the group consisting of: climacteric syndrome, osteoporosis, degenerative osteoporosis, cardiovascular and cerebrovascular diseases, and breast cancer.

According to the use of any embodiment of the first aspect, the daily dose of the medicament relative to per kg of body weight of the mammal is equivalent to 0.01-10g, preferably 0.02-5g, more preferably 0.05-1g, and further more preferably 0.05-0.5g of *Herba Cynomorii* raw material.

According to the use of any embodiment of the first aspect, the medicament comprises an extract from *Herba Cynomorii* raw material.

According to the use of any embodiment of the first aspect, the medicament comprises an extract from *Herba Cynomorii* raw material obtained by using water, an alcohol (e.g., ethanol), or a water-alcohol (e.g., ethanol) mixture (e.g., 50-98% ethanol, such as 60% ethanol, 95% ethanol) as the extracting solvent.

According to the use of any embodiment of the first aspect, the medicament comprises an extract from *Herba Cynomorii* raw material, and the extract is obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform, ethyl acetate, and n-butanol as the solvents.

According to the use of any embodiment of the first aspect, the medicament comprises an extract from *Herba Cynomorii* raw material, which extract is the petroleum ether extract, chloroform extract, ethyl acetate extract, or a mixture thereof, obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform, ethyl acetate, and n-butanol as the solvents. In an embodiment, the extract is a mixture of the petroleum ether extract, chloroform extract, and ethyl acetate extract obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform, ethyl acetate, and n-butanol as the solvents. In an embodiment, the extract is a mixture of the chloroform extract and ethyl acetate extract obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform, ethyl acetate, and n-butanol as the solvents. In an embodiment, the extract is the ethyl acetate extract obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform, ethyl acetate, and n-butanol as the solvents. In an embodiment, the extract is the petroleum ether extract obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform, ethyl acetate, and n-butanol as the solvents. In an embodiment, the extract is a mixture of the ethyl acetate extract and petroleum ether extract obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform, ethyl acetate, and n-butanol as the solvents.

According to the use of any embodiment of the first aspect, the medicament comprises an extract from *Herba Cynomorii* raw material, which extract comprises at least one of the following ingredients selected from the group consisting of: chrysophanol, emodin, catechin, (-)epicatechin-3-*O*-gallate, naringenin-4'-*O*-glucoside, and phlorizin. In an embodiment, the extract comprises the following ingredients: chrysophanol, emodin, catechin, (-)epicatechin-3-*O*-gallate, naringenin-4'-*O*-glucoside, and phlorizin.

According to the use of any embodiment of the first aspect, the medicament comprises an extract from *Herba Cynomorii* raw material, which extract comprises at least one of the following ingredients selected from the group consisting of: catechin, (-)epicatechin-3-*O*-gallate, naringenin-4'-*O*-glucoside, and phlorizin. In an embodiment, the extract comprises the following ingredients: catechin, (-)epicatechin-3-*O*-gallate, naringenin-4'-*O*-glucoside, and phlorizin. In an embodiment, the extract is ethyl acetate extract obtained by extraction using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform, ethyl acetate, and n-butanol.

According to the use of any embodiment of the first aspect, the medicament comprises an extract from *Herba Cynomorii* raw material, which extract of *Herba Cynomorii* is prepared by a process comprising the following steps: extracting *Herba Cynomorii* with an aqueous ethanol solution (e.g., 50-98% ethanol, such as 60% ethanol, 95% ethanol); extracting the obtained alcohol extract with petroleum ether, and concentrating the ether layer to obtain a petroleum ether portion; extracting the residue of petroleum ether extraction with chloroform, and concentrating the chloroform layer to obtain a chloroform portion; extracting the residue of chloroform extraction with ethyl acetate, and concentrating the ethyl acetate layer to obtain an ethyl acetate portion; extracting the residue of ethyl acetate extraction with n-butanol, and concentrating the n-butanol layer to obtain a n-butanol portion; and combining the above petroleum ether portion, chloroform portion and ethyl acetate portion to obtain the extract. In an embodiment, the *Herba Cynomorii* extract is obtained by combining the above petroleum portion and chloroform portion. In an embodiment, the *Herba Cynomorii* extract is obtained by combining the above chloroform portion and ethyl acetate portion. In an embodiment, the *Herba Cynomorii* extract is obtained by combining the above petroleum ether portion and ethyl acetate portion. In an embodiment, the *Herba Cynomorii* extract is the above ethyl acetate portion.

The first aspect of the present invention provides a *Herba Cynomorii* extract, which comprises catechin, (-)epicatechin-3-*O*-gallate, naringenin-4'-*O*-glucoside, and phlorizin, and optionally chrysophanol and/or emodin. In an embodiment, the *Herba Cynomorii* extract comprises chrysophanol, emodin, catechin, (-)epicatechin-3-*O*-gallate, naringenin-4'-*O*-glucoside, and phlorizin. In an embodiment, the *Herba Cynomorii* extract comprises catechin, (-)epicatechin-3-*O*-gallate, naringenin-4'-*O*-glucoside, and phlorizin.

According to any embodiment of the first aspect, the present invention provides a *Herba Cynomorii* extract, in which the total amount of chrysophanol, emodin, catechin, (-)epicatechin-3-*O*-gallate, naringenin-4'-*O*-glucoside, and phlorizin is 1-90%(w/w), preferably 1∼80%(w/w), preferably 2 - 50 %(w/w), preferably 2 - 40 %(w/w), preferably 2 - 30 %(w/w), and more preferably 2 - 20 %(w/w) relative to the total amount of the extract.

According to any embodiment of the first aspect, the present invention provides a *Herba Cynomorii* extract, which is an extract obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform, and ethyl acetate as the solvents.

According to any embodiment of the first aspect, the present invention provides a *Herba Cynomorii* extract, which is the ethyl acetate extract alone, or combined with the petroleum ether extractand/or the chloroform extract, , obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform and ethyl acetate as the solvents. In an embodiment, the extract is a mixture of the petroleum ether extract, chloroform extract and ethyl acetate extract obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform and ethyl acetate as the solvents. In an embodiment, the extract is a mixture of the chloroform extract and ethyl acetate extract obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform and ethyl acetate as the solvents. In an embodiment, the extract is the ethyl acetate extract obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform and ethyl acetate as the solvents.. In an embodiment, the extract is a mixture of the ethyl acetate extract and petroleum ether extract obtained by extraction using water, an alcohol (e.g., ethanol), or a water-alcohol (e.g., ethanol) mixture (e.g., 50-98% ethanol, such as 60% ethanol, 95% ethanol), and sequentially petroleum ether, chloroform and ethyl acetate as the solvents. According to any aspect of the present invention, the extract is the ethyl acetate extract and comprises catechin, naringenin-4'-*O*-glucoside, (-)epicatechin-3-*O*-gallate, and phlorizin. According to any aspect of the present invention, the extract is a mixture of the ethyl acetate extract and petroleum ether extract, and comprises catechin, naringenin-4'-*O*-glucoside, (-)epicatechin-3-*O*-gallate, phlorizin, chrysophanol.. According to any aspect of the present invention, the extract is the ethyl acetate extract, or a mixture of ethyl acetate extract and the petroleum ether extract, and comprises the ingredients catechin, (-)epicatechin-3-*O*-gallate, naringenin-4'-*O*-glucoside, phlorizin, and optionally chrysophanol and the total amount of the chrysophanol, catechin, (-)epicatechin-3-*O*-gallate, naringenin-4'-*O*-glucoside and phlorizin relative to the total amount of the extraction is 1-90%(w/w), preferably 1-80%(w/w), preferably 2 - 50 %(w/w), preferably 2 - 40 %(w/w), preferably 2 - 30 %(w/w), and more preferably 2 - 20 %(w/w).

The *Herba Cynomorii* extract according to any embodiment of the first aspect of the present invention is prepared by a process comprising the following steps: extracting *Herba Cynomorii* with an aqueous ethanol solution (e.g., 50-98% ethanol, such as 60% ethanol, 95% ethanol); extracting the obtained alcohol extract with petroleum ether, and concentrating the ether layer to obtain a petroleum ether portion; extracting the residue of the petroleum ether extraction with chloroform, and concentrating the chloroform layer to obtain a chloroform portion; extracting the residue of chloroform extraction with ethyl acetate, and concentrating the ethyl acetate layer to obtain an ethyl acetate portion; and combining the above petroleum ether portion, chloroform portion and ethyl acetate portion to obtain the extract. In an embodiment, said *Herba Cynomorii* extract is obtained by combining the above chloroform portion and ethyl acetate portion. In an embodiment, the *Herba Cynomorii* extract is obtained by combining the above petroleum ether portion and ethyl acetate portion. In an embodiment, the *Herba Cynomorii* extract is the above ethyl acetate portion.

The *Herba Cynomorii* extract according to any embodiment of the first aspect of the present invention is prepared by a process comprising the following steps: a) dipping *Herba Cynomorii* in 60-98% (preferably 70-98%, and more preferably 80-98%, such as 80%, 85%, 90%, 95% or 98%) ethanol at a volume of 2-20 times (preferably 5-15 times, and more preferably 8-12 times, such as 10 times) of the volume of *Herba Cynomorii* for 5-24 h (preferably 5-18 h, and more preferably 8-15 h); b) extracting under refluxing for 0.5-10 h (preferably 0.5-8 h, more preferably 0.5-6 h, more preferably 0.5-4 h, and further more preferably 1-4 h, such as 1 h, 2 h, 3 h, 4 h), and decanting the extract solution; c) optionally repeatedly subjecting the residue of step b) to step a) and step b) for 1-3 times (such as once, twice, 3 times); d) combining the extract solutions of step b) and step c), filtrating and recovering ethanol from the filtrate to obtain a concentrated solution; and e) extracting the concentrated solution of step d) with petroleum ether, chloroform, and ethyl acetate in that order, recovering the solvents from each of the extracts to obtain a solid, and combining the solids from the ethyl acetate portion with the petroleum portion, chloroform portion to obtain the extract. In an embodiment, the *Herba Cynomorii* extract is obtained by combining the above chloroform portion and ethyl acetate portion. In an embodiment, the *Herba Cynomorii* extract is obtained by combining the above petroleum portion and the ethyl acetate portion. In an embodiment, the *Herba Cynomorii* extract is the above ethyl acetate portion.

The second aspect of the present invention provides one or more active ingredients selected from the group consisting of: chrysophanol, emodin, catechin, (-)epicatechin-3-*O*-gallate, naringenin-4'-*O*-glucoside, and phlorizin, which are obtainable from *Herba Cynomorii.* In an embodiment, the *Herba Cynomorii* active ingredients catechin, (-)epicatechin-3-*O*-gallate, naringenin-4'-*O*-glucoside, and phlorizin,, and optionally chrysophanol and/or emodin may be used for the treatment and/or prophylaxis of a disease associated with insufficient secretion of estrogens in a mammal (e.g., human)..

For the active ingredients according to any embodiment of the second aspect, one or more of the ingredients chrysophanol, emodin, catechin, (-)epicatechin-3-*O*-gallate, naringenin-4'-*O*-glucoside, and phlorizin are obtained from *Herba Cynomorii* via extraction.

For the active ingredients according to any embodiment of the second aspect of the present invention, the steps for extracting chrysophanol, emodin, catechin, (-)epicatechin-3-*O*-gallate, naringenin-4'-*O*-glucoside, and phlorizin from *Herba Cynomorii* are as follows:
extracting *Herba Cynomorii* with an aqueous ethanol solution (e.g., 50-98% ethanol, such as 60% ethanol, or 95% ethanol);
extracting the obtained alcohol extract with petroleum ether, chloroform and ethyl acetate in that order, and recovering the solvent from each of the extracts respectively to obtain a solid of the extract for each of the extracting solvents;
subjecting the petroleum ether portion to a silica gel column chromatography, eluting with a gradient of petroleum ether-ethyl acetate solvent system, and subjecting the fractions 3-4 to recrystallization from petroleum ether to obtain chrysophanol;
subjecting the chloroform portion to a silica gel column chromatography, eluting with a gradient of petroleum ether-acetone solvent system, and subjecting the fractions 19-32 to a silica gel column chromatography with hydroxypropyl dextrane gel and a chloroform-methanol solvent system more times to obtain emodin;
subjecting the ethyl acetate portion to a silica gel column chromatography, eluting with a gradient of chloroform-methanol solvent system to obtain fractions 27-42 of the ethyl acetate portion, and fractions 43-64 of the ethyl acetate portion;
subjecting the fractions 27-42 of the ethyl acetate portion to a silica gel column chromatography, eluting with a gradient of chloroform-methanol-water solvent system to separately obtain fractions 27-42-4-6 (i.e., the fractions 4-6 obtained by subjecting the fractions 27-42 of the ethyl acetate portion from the above step to the silica gel column chromatography, similar expressions used herein in other places have a similar meaning), fractions 27-42-7-10, and fractions 27-42-11-14;
subjecting the fractions 27-42-4-6 to a silica gel column chromatography with a petroleum ether-ethyl acetate solvent system to obtain catechin;
subjecting the fractions 27-42-7-10 to a silica gel column chromatography and a reversed phase silica gel column chromatography to obtain naringenin-4'-*O*-glucoside;
subjecting the fractions 27-42-11-14 to a hydroxypropyl dextrane gel and a reversed phase silica gel column chromatography to obtain (-)epicatechin-3-*O*-gallate; and
subjecting the fractions 43-64 of the ethyl acetate portion to a polyamide and silica gel column chromatography with a chloroform-methanol solvent system in combination with a hydroxypropyl dextrane gel column chromatography to obtain phlorizin.

The third aspect of the present invention provides the use of a *Herba Cynomorii* extract in the manufacture of a medicament for the treatment and/or prophylaxis of a disease associated with insufficient secretion of estrogens in a mammal (e.g., human).

According to the use of any embodiment of the third aspect of the present invention, the disease associated with insufficient secretion of estrogens is selected from the group consisting of: climacteric syndrome, osteoporosis, degenerative osteoporosis, cardiovascular and cerebrovascular diseases, and breast cancer.

According to the use of any embodiment of the third aspect of the present invention, the daily dose of the extract for the mammal relative to per kg of body weight is equivalent to 0.01-10g, preferably 0.02-5g, more preferably 0.05-1g, and further more preferably 0.05-0.5g of *Herba Cynomorii* raw material.

According to the use of any embodiment of the third aspect of the present invention, the extract comprises the ingredients catechin, (-)epicatechin-3-*O*-gallate, naringenin-4'-*O*-glucoside, and phlorizin, and optionally chrysophanol and/or emodin,. In an embodiment, the total amount of the chrysophanol, emodin, catechin, (-)epicatechin-3-O-gallate, naringenin-4'-*O*-glucoside, and phlorizin relative to the total weight of the extract is 1-90%(w/w), preferably 1-80%(w/w), preferably 2 - 50 %(w/w), preferably 2 - 40 %(w/w), preferably 2 - 30 %(w/w), more preferably 2 - 20 %(w/w).

According to the use of any embodiment of the third aspect of the present invention, the extract comprises the following ingredients: catechin, (-)epicatechin-3-*O*-gallate, naringenin-4'-*O*-glucoside, and phlorizin. In an embodiment, the total amount of the catechin, (-)epicatechin-3-O-gallate, naringenin-4'-*O*-glucoside, phlorizin relative to the total amount of the extract is 1-90%(w/w), preferably 1-80%(w/w), preferably 2 - 50 %(w/w), preferably 2 - 40 %(w/w), preferably 2 - 30 %(w/w), more preferably 2 - 20 %(w/w).

According to the use of any embodiment of the third aspect of the present invention, the extract is obtained by extraction using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform, and ethyl acetate.

In an embodiment, the extract is a mixture of petroleum ether extract, chloroform extract, and ethyl acetate extract obtained by extraction using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform and ethyl acetate. In an embodiment, the extract is a mixture of chloroform extract and ethyl acetate extract via extraction by using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform and ethyl acetate. In an embodiment, the extract is ethyl acetate extract obtained by extraction using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform and ethyl acetate.. In an embodiment, the extract is a mixture of ethyl acetate extract and petroleum ether extract using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform and ethyl acetate.

According to the use of any embodiment of the third aspect of the present invention, the extract comprises the ingredients catechin, (-)epicatechin-3-*O*-gallate, naringenin-4'-*O*-glucoside, phlorizin, and chrysophanol. In an embodiment, the extract comprises the following ingredients: chrysophanol, emodin, catechin, (-)epicatechin-3-*O*-gallate, naringenin-4'-*O*-glucoside, and phlorizin.

According to the use of any embodiment of the third aspect of the present invention, the extract comprises the ingredients catechin, (-)epicatechin-3-*O*-gallate, naringenin-4'-*O*-glucoside, and phlorizin.. In an embodiment, the extract is ethyl acetate extract obtained by extraction using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform and ethyl acetate.

According to the use of any embodiment of the third aspect of the present invention, the *Herba Cynomorii* extract is prepared by a process comprising the following steps: extracting *Herba Cynomorii* with an aqueous ethanol solution (e.g., 50-98% ethanol, such as 60% ethanol, 95% ethanol); extracting the obtained alcohol extract with petroleum ether, and concentrating the ether layer to obtain a petroleum ether portion; extracting the residue of petroleum ether extraction with chloroform, and concentrating the chloroform layer to obtain a chloroform portion; extracting the residue of chloroform extraction with ethyl acetate, and concentrating the ethyl acetate layer to obtain an ethyl acetate portion; and combining the above petroleum ether portion and chloroform portion and ethyl acetate portion to obtain the extract. In an embodiment, the *Herba Cynomorii* extract is obtained by combining the above chloroform portion and ethyl acetate portion. In an embodiment, the *Herba Cynomorii* extract is obtained by combining the above petroleum ether portion, ethyl acetate portion. In an embodiment, the *Herba Cynomorii* extract is the above ethyl acetate portion.

According to the use of any embodiment of the third aspect of the present invention, the *Herba Cynomorii* extract is prepared by a process comprising the following steps: a) dipping *Herba Cynomorii* in 2-20 times (preferably 5-15 times, more preferably 8-12 times, such as 10 times) volume of 60-98% (preferably 70-98%, more preferably 80-98%, such as 80%, 85%, 90%, 95% or 98%) ethanol for 5-24 h (preferably 5-18 h, more preferably 8-15 h); b) extracting under refluxing for 0.5-10 h (preferably 0.5-8 h, more preferably 0.5-6 h, more preferably 0.5-4 h, more preferably 1-4 h, such as 1 h, 2 h, 3 h, 4 h), decanting the extract solution; c) optionally subjecting the residue of step b) to step a) and step b) repeatedly for 1-3 times (such as once, twice, 3 times); d) combining the extract solutions of step b) and step c), filtrating, recovering ethanol from filtrate to obtain a concentrated solution; e) subjecting the concentrated solution of step d) to petroleum ether, chloroform and ethyl acetate extraction in that order, recovering the solvent from each of extracts to obtain solids, combining the solids of any one or more of petroleum ether portion, chloroform portion, and ethyl acetate portion to obtain the extract. In an embodiment, the *Herba Cynomorii* extract is obtained by combining the above chloroform portion and ethyl acetate portion. In an embodiment, the *Herba Cynomorii* extract is obtained by combining petroleum ether portion and ethyl acetate portion. In an embodiment, the *Herba Cynomorii* extract is the above ethyl acetate portion.

The fourth aspect of the present invention provides the use of ingredients in the manufacture of a medicament for the treatment and/or prophylaxis of a disease associated with insufficient secretion of estrogens in a mammal (e.g., human), in which the ingredients are catechin, (-)epicatechin-3-*O*-gallate, naringenin-4'-*O*-glucoside, and phlorizin, and optionally chrysophanol and/or emodin,.. In an embodiment, a use of catechin, (-)epicatechin-3-*O*-gallate, naringenin-4'-*O*-glucoside and phlorizin in the manufacture of a medicament for the treatment and/or prophylaxis of a disease associated with insufficient secretion of estrogens in a mammal (e.g., human) is provided. In an embodiment, ingredients in the manufacture of a medicament for the treatment and/or prophylaxis of a disease associated with insufficient secretion of estrogens in a mammal (e.g., human) are provided, in which the ingredients are catechin, (-)epicatechin-3-*O*-gallate, naringenin-4'-*O*-glucoside, and phlorizin.

According to the use of any one item of the fourth aspect of the present invention, the disease associated with insufficient secretion of estrogens is selected from the group consisting of: climacteric syndrome, osteoporosis, degenerative osteoporosis, cardiovascular and cerebrovascular diseases, and breast cancer.

The use according to any one item of the fourth aspect of the present invention is the use of the following ingredients in the manufacture of a medicament for the treatment and/or prophylaxis of a disease associated with insufficient secretion of estrogens in a mammal (e.g., human), which ingredients are catechin, (-)epicatechin-3-*O*-gallate, naringenin-4'-*O*-glucoside, and phlorizin, and optionally chrysophanol and/or emodin.

According to the use of any one item of the fourth aspect of the present invention, the ingredients of chrysophanol, emodin, catechin, (-)epicatechin-3-*O*-gallate, naringenin-4'-*O*-glucoside, and phlorizin are obtained via extraction from *Herba Cynomorii.*

According to the use of any one item of the fourth aspect of the present invention, the steps for extracting chrysophanol, emodin, catechin, (-)epicatechin-3-*O*-gallate, naringenin-4'-*O*-glucoside, and phlorizin from *Herba Cynomorii* are as follows:
extracting *Herba Cynomorii* with an aqueous ethanol solution (e.g., 50-98% ethanol, such as 60% ethanol, 95% ethanol);
subjecting the obtained alcohol extract to petroleum ether, chloroform and ethyl acetate extraction in that order, and recovering the solvent respectively from each of the extracts to obtain a solid of the extract for each of the extracting solvents;
subjecting the petroleum ether portion to a silica gel column chromatography, eluting with a gradient of petroleum ether-ethyl acetate solvent system, and subjecting the fractions 3-4 to recrystallization from petroleum ether to obtain chrysophanol;
subjecting the chloroform portion to a silica gel column chromatography, eluting with a gradient of petroleum ether-acetone solvent system, and subjecting repeatedly the fractions 19-32 to a silica gel column chromatography with hydroxypropyl dextrane gel and a chloroform-methanol solvent system to obtain emodin;
subjecting the ethyl acetate portion to a silica gel column chromatography, eluting with a gradient of chloroform-methanol solvent system to obtain fractions 27-42 of the ethyl acetate portion, and fractions 43-64 of the ethyl acetate portion;
subjecting the fractions 27-42 of the ethyl acetate portion to a silica gel column chromatography, eluting with a gradient of chloroform-methanol-water solvent system, to separately obtain fractions 27-42-4-6 (i.e., the fractions 4-6 obtained from the fractions 27-42 of the ethyl acetate portion from the last step by a silica gel column chromatography, similar expressions used herein in other places have a similar meaning), fractions 27-42-7-10, and fractions 27-42-11-14;
subjecting the fractions 27-42-4-6 to a silica gel column chromatography with a petroleum ether-ethyl acetate solvent system to obtain catechin;
subjecting the fractions 27-42-7-10 to a silica gel column chromatography and a reversed phase silica gel column chromatography to obtain naringenin-4'-*O*-glucoside;
subjecting the fractions 27-42-11-14 to a hydroxypropyl dextrane gel chromatography and a reversed phase silica gel column chromatography to obtain (-)epicatechin-3-*O*-gallate;
subjecting the fractions 43-64 of the ethyl acetate portion to a polyamide and silica gel column chromatography with a chloroform-methanol solvent system in combination with a hydroxypropyl dextrane gel column chromatography to obtain phlorizin.

The fifth aspect of the present invention provides the use of *Herba Cynomorii* in the manufacture of a medicament as phytoestrogen.

According to the use of any embodiment of the fifth aspect of the present invention, the medicament may be used for the treatment and/or prophylaxis of climacteric syndrome, osteoporosis, degenerative osteoporosis, cardiovascular and cerebrovascular diseases, and breast cancer.

According to the use of any embodiment of the sixth aspect of the present invention, the daily dose of the medicament for the mammal relative to per kg of body weight is equivalent to 0.01-10g, preferably 0.02-5g, more preferably 0.05-1 g, and further more preferably 0.05-0.5g of *Herba Cynomorii* raw material.

According to the use of any embodiment of the fifth aspect of the present invention, the medicament comprises an extract from *Herba Cynomorii* raw material.

According to the use of any embodiment of the fifth aspect of the present invention, the medicament comprises an extract from *Herba Cynomorii* raw material, which extract is obtained by extraction using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform and ethyl acetate.

According to the use of any embodiment of the fifth aspect of the present invention, the medicament comprises an extract from *Herba Cynomorii* raw material, which extract is the ethyl acetate extract alone, or combined with the petroleum ether extract, and/or the chloroform extract, obtained by extraction using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform and ethyl acetate. In an embodiment, the extract is a mixture of petroleum ether extract, chloroform extract, and ethyl acetate extract obtained by extraction using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform and ethyl acetate. In an embodiment, the extract is a mixture of chloroform extract and ethyl acetate extract obtained by extraction using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform and ethyl acetate. In an embodiment, the extract is ethyl acetate extract obtained by extraction using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform and ethyl acetate. In an embodiment, the extract is a mixture of ethyl acetate extract and petroleum ether extract via extraction by using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform and ethyl acetate.

According to the use of any embodiment of the fifth aspect of the present invention, the medicament comprises an extract from *Herba Cynomorii* raw material, which extract comprises catechin, (-)epicatechin-3-*O*-gallate, naringenin-4'-*O*-glucoside, and phlorizin, and optionally chrysophanol and/or emodin,. In an embodiment, the extract comprises the following ingredients: chrysophanol, emodin, catechin, (-)epicatechin-3-O-gallate, naringenin-4'-*O*-glucoside, and phlorizin.

According to the use of any embodiment of the fifth aspect of the present invention, the medicament comprises an extract from *Herba Cynomorii* raw material, which extract comprises the ingredients catechin, (-)epicatechin-3-*O*-gallate, naringenin-4'-*O*-glucoside, and phlorizin. In an embodiment, the extract is ethyl acetate extract obtained by extraction using water, an alcohol (e.g., ethanol), or a water-alcohol (e.g., ethanol) mixture (e.g., 50-98% ethanol, such as 60% ethanol, 95% ethanol) as the solvent, and sequentially petroleum ether, chloroform and ethyl acetate.

According to the use of any embodiment of the fifth aspect of the present invention, the medicament comprises an extract from *Herba Cynomorii* raw material, which extract of *Herba Cynomorii* is prepared by a process comprising the following steps: extracting *Herba Cynomorii* with an aqueous ethanol solution (e.g., 50-98% ethanol, such as 60% ethanol, 95% ethanol); extracting the obtained alcohol extract with petroleum ether, and concentrating the ether layer to obtain a petroleum ether portion; extracting the residue of petroleum ether extraction with chloroform, and concentrating the chloroform layer to obtain a chloroform portion; extracting the residue of chloroform extraction with ethyl acetate, and concentrating the ethyl acetate layer to obtain an ethyl acetate portion; and combining the above petroleum ether portion, chloroform portion and ethyl acetate portion to obtain the extract. In an embodiment, the *Herba Cynomorii* extract is obtained by combining the above chloroform portion and ethyl acetate portion. In an embodiment, the *Herba Cynomorii* extract is obtained by combining the above petroleum ether portion and ethyl acetate portion. In an embodiment, the *Herba Cynomorii* extract is the above ethyl acetate portion.

In an embodiment of the use according to any embodiment of the fifth aspect of the present invention, the extract has the features of the extract of the first aspect or the second aspect of the present invention.

The sixth aspect of the present invention provides the use of a *Herba Cynomorii* extract in the manufacture of a medicament as phytoestrogen.

According to the use of any embodiment of the sixth aspect of the present invention, the medicament may be used for the treatment and/or prophylaxis of climacteric syndrome, osteoporosis, degenerative osteoporosis, cardiovascular and cerebrovascular diseases, and breast cancer.

According to the use of any embodiment of the sixth aspect of the present invention, the daily dose of the medicament for the mammal relative to per kg of body weight is equivalent to 0.01-10g, preferably 0.02-5g, more preferably 0.05-1 g, more preferably 0.05-0.5g of *Herba Cynomorii* raw material.

According to the use of any embodiment of the sixth aspect of the present invention, the extract has the features of the extract according to any embodiment of the first aspect of the present invention.

The seventh aspect of the present invention provides the use of the following ingredients in the manufacture of a medicament as phytoestrogen: catechin, (-)epicatechin-3-*O*-gallate, naringenin-4'-*O*-glucoside, and phlorizin, and optionally chrysophanol and/or emodin,.

According to the use of any embodiment of the seventh aspect of the present invention, the medicament may be used for the treatment and/or prophylaxis of climacteric syndrome, osteoporosis, degenerative osteoporosis, cardiovascular and cerebrovascular diseases, and breast cancer.

The use according to any embodiment of the seventh aspect of the present invention is a use of the following ingredients in the manufacture of a medicament as phytoestrogen: chrysophanol, emodin, catechin, (-)epicatechin-3-*O*-gallate, naringenin-4'-*O*-glucoside, and phlorizin.

According to the use of any embodiment of the eighth aspect of the present invention, the chrysophanol, emodin, catechin, (-)epicatechin-3-*O*-gallate, naringenin-4'-*O*-glucoside, phlorizin independently have the features of any embodiment of the second aspect of the present invention.

The eighth aspect of the present invention provides a pharmaceutical composition for the treatment and/or prophylaxis of a disease associated with insufficient secretion of estrogens in a mammal (e.g., human), which comprises a therapeutically and/or prophylactically effective amount of *Herba Cynomorii* extract and optionally a pharmaceutically acceptable excipient.

According to the pharmaceutical composition of any embodiment of the eighth aspect of the present invention, the *Herba Cynomorii* extract comprises the ingredients catechin, (-)epicatechin-3-*O*-gallate, naringenin-4'-*O*-glucoside, and phlorizin, and optionally chrysophanol and/or emodin,.

According to the pharmaceutical composition of any embodiment of the eighthaspect of the present invention, the *Herba Cynomorii* extract comprises the following ingredients: chrysophanol, emodin, catechin, (-)epicatechin-3-*O*-gallate, naringenin-4'-*O*-glucoside, and phlorizin.

According to the pharmaceutical composition of any embodiment of the ninth aspect of the present invention, the total amount of the chrysophanol, emodin, catechin, (-)epicatechin-3-*O*-gallate, naringenin-4'-*O*-glucoside, and phlorizin relative to the total weight of the extract is 1-90%(w/w), preferably 1-80%(w/w), preferably 2 - 50 %(w/w), preferably 2 - 40 %(w/w), preferably 2 - 30 %(w/w), and more preferably 2 - 20 %(w/w).

According to the pharmaceutical composition of any embodiment of the eighth aspect of the present invention, the daily dose of the pharmaceutical composition administered to the mammal relative to per kg of body weight is equivalent to 0.01-10g, preferably 0.02-5g, more preferably 0.05-1g, and further more preferably 0.05-0.5g of *Herba Cynomorii* raw material.

According to the pharmaceutical composition of any embodiment of the ninth aspect of the present invention, the *Herba Cynomorii* extract is prepared by a process comprising the following steps: extracting with water, an alcohol (e.g., ethanol), or a water-alcohol (e.g., ethanol) mixture (e.g., 50-98% ethanol, such as 60% ethanol, 95% ethanol) as the solvent, and subjecting to petroleum ether, chloroform and ethyl acetate extraction in order to obtain a petroleum ether extract, chloroform extract, ethyl acetate extract, or a mixture thereof.

According to the pharmaceutical composition of any embodiment of the eighthaspect of the present invention, the *Herba Cynomorii* extract has the features of the extract according to any embodiment of the second aspect of the present invention.

The ninth aspect of the present invention provides a pharmaceutical composition for the treatment and/or prophylaxis of a disease associated with insufficient secretion of estrogens in a mammal (e.g., human), which comprises a therapeutically and/or prophylactically effective amount of the pharmaceutically active ingredients catechin, (-)epicatechin-3-*O*-gallate, naringenin-4'-*O*-glucoside, and phlorizin, an optionally chrysophanol and/or emodin and optionally a pharmaceutically acceptable excipient,.

The pharmaceutical composition according to any embodiment of the ninth aspect of the present invention, comprises a therapeutically and/or prophylactically effective amount of chrysophanol, emodin, catechin, (-)epicatechin-3-*O*-gallate, naringenin-4'-*O*-glucoside, phlorizin, and optionally a pharmaceutically acceptable excipient.

The pharmaceutical composition according to any embodiment of the ninth aspect of the present invention, comprises a therapeutically and/or prophylactically effective amount of catechin, (-)epicatechin-3-*O*-gallate, naringenin-4'-*O*-glucoside, phlorizin, and optionally a pharmaceutically acceptable excipient.

According to the pharmaceutical composition of any embodiment of the tenth aspect of the present invention, the chrysophanol, emodin, catechin, (-)epicatechin-3-*O*-gallate, naringenin-4'-*O*-glucoside, and phlorizin independently have the features of any embodiment of the second aspect of the present invention.

The tenth aspect of the present invention provides a method for the treatment and/or prophylaxis of a disease associated with insufficient secretion of estrogens in a subject in need thereof, which comprises administering to the subject a therapeutically and/or prophylactically effective amount of *Herba Cynomorii* extract. In an embodiment, the *Herba Cynomorii* is administered to the subject in a form of medicament.

The tenth aspect of the present invention further provides a method for the treatment and/or prophylaxis of a disease associated with insufficient secretion of estrogens in a subject in need thereof, which comprises administering to the subject a therapeutically and/or prophylactically effective amount of the *Herba Cynomorii* extract according to any embodiment of the first aspect of the present invention.

The tenth aspect of the present invention further provides a method for the treatment and/or prophylaxis of a disease associated with insufficient secretion of estrogens in a subject in need thereof, which comprises administering to the subject a therapeutically and/or prophylactically effective amount of the following ingredients: catechin, (-)epicatechin-3-*O*-gallate, naringenin-4'-*O*-glucoside, and phlorizin, and optionally chrysophanol and/or emodin,. In an embodiment, the chrysophanol, emodin, catechin, (-)epicatechin-3-*O*-gallate, naringenin-4'-*O*-glucoside, and phlorizin independently have the features of any embodiment of the second aspect of the present invention.

According to the method of any embodiment of the eleventh aspect of the present invention, the disease associated with insufficient secretion of estrogens is selected from the group consisting of: climacteric syndrome, osteoporosis, degenerative osteoporosis, cardiovascular and cerebrovascular diseases, and breast cancer.

The features of any aspects of the present invention or any embodiment of the any aspects are also suitable for any other aspect or any embodiment of the any other aspect, as long as they are not contradictory between each other, and necessary modifications may be applied when they are compatible. In the present invention, for example, when "any embodiment of the first aspect of the present invention" is mentioned, the "any item" refers to any sub-aspect of the first aspect of the present invention; and similar expressions for other aspects have the same meanings.

The aspects and features of the present invention are further described as follows.

All references cited are incorporated herein in their entirity by reference, and if any expressions in these documents have different meanings from those in the present invention, the expressions of the present invention should prevail. In addition, the terms and phrases used in the present invention have general meanings well known by those skilled in the art, unless specified and explained otherwise in the present invention, and when the mentioned terms and phrases have meanings inconsistent to their known meanings, the meanings described in the present invention should prevail.

The term *"Herba Cynomorii"* used herein meets the definition under entry *"Herba Cynomorii"* ( 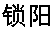 ) of the First Part of the Pharmacopoeia of the People's Republic of China, 2005 Edition.

The term "about" used herein generally refers to allowable error range in the art, such as ±10%, such as ±5%, such as ±2%.

As used herein, the term "effective amount" refers to a dose that may fulfill treatment, prophylaxis, alleviation and/or relief of the disease or disorder in a subject as mentioned in the present invention.

As used herein, the term "pharmaceutical composition" may interchange for "composition", and refers to a substance that may fulfill treatment, prophylaxis, alleviation and/or relief of the disease or disorder in a subject as mentioned in the present invention.

As used herein, the term "subject" or "patient" refers to an animal, especially a mammal, such as human, dog, monkey, cattle, horse, etc., which is administered with the composition and extract for treatment, prophylaxis, alleviation and/or relief of the disease or disorder as mentioned in the present invention.

As used herein, the term "disease or disorder" refers to a physical state of the subject, the physical state associates with the disease or disorder as mentioned in the present invention.

As used herein, unless specifically indicated otherwise, "%" generally refers to a weight/weight percentage when total matter is solid, and generally refers to a weight/volume percentage when total matter is liquid. Of course, when total matter is liquid and solute is liquid, the percentage for characterizing the liquid solute generally refers to volume/volume percentage. As for % used for describing ethanol, such as 95% ethanol, it has a meaning well known in the art, for example, the definition in the guide section of the Second Part of Pharmacopoeia of the People's Republic of China, 2005 Edition.

The phrase "disease associated with insufficient secretion of estrogens" in the text has the meaning well known by those skilled in the art, and generally refers to diseases or disorders induced by insufficient secretion of estrogens in a female mammal, such as a human, especially, a climacteric woman, and these diseases or disorders include but are not limited to climacteric syndrome, osteoporosis, degenerative osteoporosis, cardiovascular and cerebrovascular diseases, and breast cancer.

As used herein, if not specifically indicated, the term "extract" refers to the "extract" from *Herba Cynomorii.*

As used herein, the term "catechin" may refer to (-)catechin, (+)catechin, or racemic catechin.

As used herein, the term "petroleum ether portion" refers to an effective site of plant obtained by petroleum ether treatment (extraction), and may also be called as "petroleum ether site". Other similar terms such as "ethyl acetate portion" also have similar meanings, i.e., for example, effective site of plant obtained by liquid-liquid extraction.

Although the present invention describes the mentioned active ingredients in detail, the inventors of the present invention still further emphasize that these active ingredients (which include, but are not limited to, chrysophanol, emodin, catechin, (-)epicatechin-3-*O*-gallate, naringenin-4'-*O*-glucoside, phlorizin) have chemical structure, physical and chemical properties as known by those skilled in the art.

According to the present invention, since 6 ingredients, chrysophanol, emodin, catechin, (-)epicatechin-3-*O*-gallate, naringenin-4'-*O*-glucoside, phlorizin, all have corresponding phytoestrogen functions, the amounts of these 6 ingredients or portions thereof in extract, medicament, composition (e.g., proportion of each of ingredients relative to total extract, medicament, composition, proportion between ingredients, and so on) are not specifically defined, and the dose of medicament may be calculated by conversion into *Herba Cynomorii* raw material.

A series of compounds obtained by separation are used in estrogen receptor activation test, in which estradiol is used as positive control, and all of chrysophanol, emodin, catechin, (-)epicatechin-3-*O*-gallate, naringenin-4'-*O*-glucoside, phlorizin show relevance to estrogen receptor activation.

Conventional techniques in the pharmaceutical field may be used to mix single active ingredients of *Herba Cynomorii* or a combination thereof or an *Herba Cynomorii* extract containing estrogen active ingredients in *Herba Cynomorii* with one or more pharmaceutically acceptable excipients, and form a desired dosage form, so as to prepare the pharmaceutical composition of the present invention.

The term "pharmaceutically acceptable excipient" used in the pharmaceutical composition may be any conventional excipients in the pharmaceutical field. The selection of specific excipients depends on manner of administration or disease type and state of a specific patient to be treated. Manufacture methods of pharmaceutical compositions suitable for specific administration manners are known in the art. For example, pharmaceutically acceptable excipients include conventional diluents, carriers, fillings, bindings, wetting agent, disintegrants, absorption enhancers, surfactants, absorption supports and lubricants in the pharmaceutical field. If necessary, flavors, preservatives and sweetening agents may be added to the pharmaceutical compositions.

The pharmaceutical composition of the present invention may be processed as tablets, powders granules, capsules, oral liquids, pastes, creams, or injection emulsions (aseptic powder for injection). The above dosage forms may be prepared according to conventional methods in the pharmaceutical field.

The studies show that the compounds obtained in the present invention have a pseudo-estrogen function in different degree. Hence, it may be expected that the compound obtained in the present invention (e.g., chrysophanol, emodin, catechin, (-)epicatechin-3-*O*-gallate, naringenin-4'-*O*-glucoside, phlorizin) or the pharmaceutical composition comprising it may be used for treatment of climacteric syndrome, breast cancer and/or cardiovascular and cerebrovascular diseases. Or, the one obtained in the present invention may be used for manufacture of a medicament for treatment of climacteric syndrome, breast cancer and/or cardiovascular and cerebrovascular diseases.

The present invention uses the cell transfection and luciferase reporter gene chemiluminescence detection methods to study the effects of ten single compounds from *Herba Cynomorii* on transcriptional activity of ERα and ERβ, indicating which ingredients of *Herba Cynomorii* have estrogen activity, and illustrating their target points and action routes. The results surprisingly show that four flavone compounds (catechin, phlorizin, naringenin-4'-O-glucoside and (-)epicatechin-3-O-gallate have a potent activity of estrogen, both catechin (10⁻⁵M) and phlorizin (10⁻⁶-10⁻⁵M) may act as antagonists of the transcriptional activity of ERα and ERβ, and have no significant selectivity to receptor subtypes; naringenin-4'-O-glucoside (10⁻⁹-10⁻⁵M) may act as antagonists of the transcriptional activity of ERα and ERβ in a dose dependent manner, and has a higher selectivity to ERβ than ERα; while (-)epicatechin-3-O-gallate (10⁻⁸-10⁻⁵M) may act as antagonists of ERα and ERβ with the highest transcription activity at 10⁻⁷M, and has a higher selectivity to ERα than ERβ. The estrogen activity of the flavone compounds is about 1/1000-1/10 of that of estradiol. The two anthraquinone compounds from *Herba Cynomorii,* chrysophanol and emodin may activate the receptor transcription activity at the high concentration of 10⁻⁶M, have no receptor selectivity, and their activity is about 1/1000-1/100 of that of estradiol.

### EXEMPLARY MODES FOR CARRYING OUT THE INVENTION

The present invention is further illustrated by the following examples and experiments. It should be understood that these examples and experiments are merely provided for illustrating the invention, rather than restricting the invention in any way.

The materials and experimental methods used in the examples are generally and/or specifically described. Although many materials and methods used for fulfilling the present invention are well known in the art, they are still further provided with a description as detailed as possible. Those skilled in the art would clearly understand that the materials and methods used in the present invention in the text are known in the art, if they are not specifically described.

### Example 1: Preparation of the four flavone compounds and 2 anthraquinone compounds from Herba Cynomorii

### 1. Preparation of the extract

40.0 kg of *Herba Cynomorii* raw material (purchased from Alxa, Inner Mongolia) was crushed into small pieces, added with 400 liters of 95% ethanol, and immersed overnight. After extracting under refluxing for 2 h, extract solution was decanted, and the residue was added with 400 liters of 60% ethanol and extracted under refluxing for 2 h. The extract solutions were combined and filtrated, and ethanol was recovered from the filtrate via rotatory evaporation. The residue obtained after concentrating was extracted sequentially with petroleum ether, chloroform, ethyl acetate and n-butanol, and a solid was obtained after recovering the solvents, respectively. **Results:** 55 g solid from the petroleum ether portion, 229 g solid from the chloroform portion, 147 g solid from the ethyl acetate portion, and 867 g solid from the n-butanol portion were obtained. The petroleum ether portion contained chrysophanol in a mass percentage of 0.082% (W/W), the chloroform portion contained emodin in 0.031% (W/W), the ethyl acetate portion contained catechin in a mass percentage of 10.84% (W/W), the ethyl acetate portion contained naringenin-4'-*O*-glucoside in a mass percentage of 0.304% (W/W), the ethyl acetate portion contained (-)epicatechin-3-*O*-gallate in a mass percentage of 0.88% (W/W), and the ethyl acetate portion contained phlorizin in a mass percentage of 0.28% (W/W).

### 2. Preparation of individual active ingredients

55 g of the petroleum ether portion from *Herba Cynomorii* was subjected to a silica gel column chromatography, eluting with a gradient of petroleum ether-ethyl acetatesolvent system, and the fractions 3-4 were subjected to recrystallization from petroleum ether to obtain an orange red cluster crystal, 30mg, identified as chrysophanol.

229 g of the chloroform portion of *Herba Cynomorii* was subjected to a silica gel column chromatography, eluting with a gradient of petroleum ether-acetone solvent system, the fractions 19-32 were subjected to a hydroxypropyl dextrane gel chromatography and repeated silica gel column chromatography with a chloroform-methanol solvent system to obtain orange red cluster crystal, 35mg, identified as emodin.

147 g of the ethyl acetate portion *Herba Cynomorii* was subjected to a silica gel column chromatography, eluting with a gradient of chloroform-methanol solvent system to separately obtain fractions 27-42 of the ethyl acetate portion and fractions 43-64 of the ethyl acetate portion.

The fractions 27-42 of the ethyl acetate portion were subjected to a silica gel column chromatography, eluting with a gradient of chloroform-methanol-water solvent system. The obtained fractions 27-42-4-6 were subjected to a silica gel column chromatography with a petroleum ether-ethyl acetate solvent system to obtain a white cluster crystal, 13g, identified as catechin (raceme). The obtained fractions 27-42-7-10 were subjected to a silica gel column chromatography and a reversed phase silica gel column chromatography to obtain white powder, 300mg, identified as naringenin-4'-*O*-glucoside. The obtained fractions 27-42-11-14 were subjected to a hydroxypropyl dextrane gel chromatography and reversed phase silica gel column chromatography to obtain an off-white powder, 1g, identified as (-)epicatechin-3-*O*-gallate.

The fractions 43-64 of the ethyl acetate portion were subjected to a polyamide chromatography and silica gel column chromatography with a chloroform-methanol solvent system in combination with a hydroxypropyl dextrane gel column chromatography to obtain a brown needle crystal, 200mg, identified as phlorizin.

### Example 2: Confirmation of the structures of the 6 compounds from Herba Cynomorii

The structures of the 6 compounds (i.e., chrysophanol, emodin, catechin (raceme), (-)epicatechin-3-*O*-gallate, naringenin-4'-*O*-glucoside, phlorizin) obtained in Example 1 were confirmed (entrusting the Analysis Testing Center of Tianjin University to perform test, 500MHz NMR). The results of analysis show that the structures of these compounds are consistent with those reported in the references, and the specific data are as follows.

Chrysophanol, as an orange red cluster crystal. ¹H NMR (CDCl₃, 500 MHz), δ: 7.09 (1 H, br s, H-2), 7.64 (1 H, br s, H-4), 7.81 (1 H, br d, *J* = 7.5 Hz, H-5), 7.67 (1 H, br d, *J* = 8.0 Hz, H-6), 7.28 (1 H, br d, *J* = 8.5 Hz, H-7), 2.47 (3H, s, -CH₃), 12.00 (1 H, s, -OH), 12.12 (1 H, s, -OH); ¹³C NMR (CDCl₃), δ: 162.6 (C-1), 124.8 (C-2), 149.6 (C-3), 121.6 (C-4), 124.6 (C-5), 137.1 (C-6), 120.1 (C-7), 162.9 (C-8), 192.7 (C-9), 182.1 (C-10), 133.8 (C-11), 113.9 (C-12), 116.0 (C-13), 133.5 (C-14), 22.5 (-CH₃).

Emodin, as an orange red cluster crystal. ¹H NMR (DMSO-d*₆*, 500 MHz), δ: 7.06 (1 H, d, *J* = 2 Hz, H-2), 7.39 (1 H, d, *J* = 2 Hz, H-4), 7.03 (1 H, d, *J* = 3.0 Hz, H-5), 6.50 (1 H, d, *J =* 3.0 Hz, H-7), 2.36 (3H, s, C6-CH₃).

Catechin (raceme), as a white cluster crystal. ¹H NMR (CD₃OD, 500 MHz), δ: 4.56 (1 H, d, *J* = 7.5 Hz, H-2), 3.97 (1 H, m, H-3), 2.85 (1 H, dd, *J* = 16.0, 5.5 Hz, H-4a), 2.50 (1 H, dd, *J* = 16.0, 8.0 Hz, H-4b), 5.93 (1 H, d, *J =* 1.0 Hz, H-6), 5.86 (1 H, br s, H-8), 6.84 (1 H, br s, H-2'), 6.76 (1 H, d, *J* = 8.0 Hz, H-5'), 6.72 (1 H, d, *J* = 8.5 Hz, H-6'). ¹³C NMR (CD₃OD, 500 MHz), δ: 81.7 (C-2), 67.6 (C-3), 27.3 (C-4), 99.8 (C-4a), 156.4 (C-5), 95.1 (C-6), 156.6 (C-7), 94.3 (C-8), 155.7 (C-8a), 131.0 (C-1'), 114.1 (C-2'), 145.1 (C-3'), 145.1 (C-4'), 114.9 (C-5'), 118.9 (C-6'). The samples of 1.0 g/100 ml and 0.1 g/100 ml in methanol solution have specific optical rotation of zero.

(-)Epicatechin-3-*O*-gallate, as an off-white powder. ¹H NMR (CD₃OD, 500 MHz), δ: 5.03 (1 H, s, H-2), 5.53 (1 H, s, H-3), 2.99 (1 H, dd, *J* = 17.5, 4.5 Hz, H-4a), 2.85 (1 H, dd, *J* = 17.0, 1.5 Hz, H-4b), 5.96 (1 H, s, H-6), 5.96 (1 H, s, H-8), 6.93 (1 H, d, *J* = 2.0 Hz, H-2'), 6.70 (1 H, d, *J* = 7.5 Hz, H-5'), 6.81 (1 H, dd, *J* = 8.0, 2.0 Hz, H-6'), 6.95 (2H, s, H-2" and H-6"). ¹³C NMR (CD₃OD, 500 MHz), δ: 78.5 (C-2), 69.9 (C-3), 26.8 (C-4), 99.4 (C-4a), 157.7 (C-5 and C-7), 96.5 (C-6), 95.8 (C-8), 157.2 (C-8a), 131.4 (C-1'), 115.0 (C-2'), 145.8 (C-3' and C-4'), 116.0 (C-5'), 119.3 (C-6'), 121.3 (C-1 "), 110.1 (C-2" and C-6"), 146.2 (C-3" and C-5"), 139.7 (C-4"), 167.6 (C-CO).

Naringenin-4'-*O*-glucoside, as a white powder. ¹H-NMR (DMSO-*d₆*, 500 MHz), δ: 5.52 (1 H, dd, *J* = 13, 3.0 Hz, H-2), 2.72-3.20 (2H, m, H-3), 5.88 (1 H, d, *J =* 1.5 Hz, H-6), 5.87 (1 H, d, *J* = 2.0 Hz, H-8), 7.42 (2H, d, *J* = 8.5 Hz, H-2' and H-6'), 7.05 (2H, d, *J* = 9.0 Hz, H-3' and H-5'), 4.88 (1 H, d, *J* = 7.5 Hz, saccharide H-1), 3.17-4.55 (6H, m, saccharide proton), 12.13 (1 H, s, C₅-OH).

Phlorizin, as a brown needle crystal. ¹H NMR (CD₃OD, 500 MHz), δ: 7.06 (2H, d, *J* = 8.5 Hz, H-2, 6), 6.68 (2H, d, *J* = 8.5 Hz, H-3, 5), 6.18 (1 H, d, *J* = 2.0 Hz, H-3'), 5.95 (1 H, d, *J =* 2.0 Hz, H-5'), 5.04 (1 H, d, *J =* 7.0 Hz, H-1 "), 3.43-3.48 (3H, overlapped, H-2", 3", 5 "), 3.43 (1 H, m, H-4"), 3.91 (1 H, d, *J* = 2.0, 12.5 Hz, H-6a"), 3.72 (1 H, d, *J* = 5.5, 12.5 Hz, H-6b"), 3.40 (2H, t, *J* = 7.5 Hz, H-α), 2.88 (2H, t, *J* = 7.5 Hz, H-β). ¹³C NMR (CD₃OD, 500 MHz), δ: 133.8 (C-1), 130.4 (C-2, 6), 116.1 (C-3, 5), 156.3 (C-4), 46.9 (C-α), 30.8 (C-β), 106.8 (C-1'), 162.3 (C-2'), 95.4 (C-3'), 165.9 (C-4'), 98.3 (C-5'), 167.5 (C-6'), 102.2 (C-1"), 74.7 (C-2"), 78.4 (C-3"), 71.1 (C-4"), 78.4 (C-5"), 62.4 (C-6").

### Example 3: Activation of the compounds on the estrogen receptors

Cell transfection method was used to observe the selective activation of estrogen receptor by catechin, (-)epicatechin-3-*O*-gallate, naringenin-4'-*O*-glucoside, phlorizin, emodin, chrysophanol. Actually, estrogens show their biological activity by regulating estrogen responding genetic transcription.

The reporter gene-carrying ER_{α} or ER_{β}DNA fragments were separately transfected in ER expression free Hela cells, after the substance to be tested was added to cell culture medium, the reporter gene transcription activation was observed to determine whether the substance to be tested exerts estrogen function via ER_{α} or ER_{β}.

Formulation of sample solutions of catechin, (-)epicatechin-3-*O*-gallate, naringenin-4'-*O*-glucoside, phlorizin, emodin, chrysophanol (separately obtained from Example 1):

Catechin, (-)epicatechin-3-*O*-gallate, naringenin-4'-*O*-glucoside, phlorizin, emodin, chrysophanol in corresponding weights were separately weighed precisely, than added with DMSO for dissolution, the corresponding sample solution high dose groups (10µM or 1µM) were diluted in certain times to separately obtain low dose groups (1µM to 0.001 µM).

Formulation of solutions of ethyl acetate portion and petroleum ether portion:
Ethyl acetate portion (from 147g of production obtained in Example 1) and petroleum ether portion (from 55g of production obtained in Example 1) of *Herba Cynomorii* were separately weighed precisely and added with corresponding weights of DMSO for dissolution, the corresponding sample solution high dose group (50µg/ml) was diluted in certain times to separately obtain low dose groups (5 µg/ml, 0.5 µg/ml and 0.05 µg/ml).

Under condition of 37°C and 5% CO₂, Hela cells were cultured in phenol red-free high sugar DMEM culture medium containing 10% calf serum in incubator, the cells were spread in 60% density on 96-well plate when cell density was about 90%, each well was added with 150ul of phenol red-free DMEM culture medium containing 10% serum, cultivation was performed overnight. To each well of the 96-well plate, 100ul of serum-free DMEM culture medium and 0.4µg plasmid (ptk-ERE-luc 2pg, tk-Renila 1µg, ERα or ERβ 1µg) were used for transfection. After 6 h of transfection, each well was added with 150ul of serum-free phenol red-free DMEM culture medium. Three wells were selected from each group, added with 10-8M estradiol E₂, and substance to be tested in different concentrations, 15ul/well (sample 4-5 concentration gradient groups), continuously cultured under conditions of 37°C, 5%CO₂ for 24 h. After 24 h, each well was added with 80µl of cell disruption solution, and the cells were subjected to freeze thawing once to be sufficiently disrupted. As for the cell lysis solution of each well, its activities to firefly luciferase and Renila luciferase were separately measured. Each well used firefly luciferase/Renila luciferase value as result, the well without estrogen treatment in each group was used as control, the ratio of the group added with estrogen or substance to be tested to the control group was calculated, and the average value of three wells of each group was used. The results are shown in Table 1, Table 2 and Table 3.

**Table 1: Effects of the petroleum ether portion and ethyl acetate portion from Herba Cynomorii on ERα or ERβ transcription activity in Hela cells**

| Sample | Concentration (µg/ml) | ERα (% of the control) mean±S.D. (% of estradiol) | ERβ (% of the control) mean±S.D.( % of estradiol) |
|---|---|---|---|
| Ethyl acetate portion | 50 | 94.33±5.80 (44.92) | 119.21±1.86 (51.68) |
| | 5 | 90.83±7.34 (43.25) | 118.94±5.63 (49.56) |
| | 0.5 | 84.18±12.84 (40.08) | 115.32±2.46 (48.05) |
| | 0.05 | 79.275±8.57 (37.75) | 94.42±2.46 (39.34) |
| Petroleum ether portion | 50 | 73.51±8.76 (39.10) | 75.99±12.83 (38.97) |
| | 5 | 75.09±9.87 (39.94) | 59.09±15.27 (30.30) |
| | 0.5 | 54.42±9.89 (28.95) | 71.67±11.31 (36.76) |
| | 0.05 | 65.64±6.76 (34.92) | 75.99±5.91 (38.97) |

The results in the table show that the extracts of the present invention have the desired biological activity. Those skilled in the art would understand that although only the activity of the ethyl acetate portion and petroleum ether portion are indicated in the table, it may be expected that any one or more of the extracts obtained before the extraction with petroleum ether, chloroform, ethyl acetate and n-butanol and the effective portions obtained after the extraction with the four solvents would have the desired biological activity.

**Table 2: Effects of the flavone compounds on ERα or ERβ transcription activity in Hela cells**

| Sample | Concentration (M) | ERα (% of the control) mean±S.D. (% of estradiol) | ERβ (% of the control) mean±S.D. (% of estradiol) | ERβ/ERα (both % of estradiol at 10⁻⁸M) |
|---|---|---|---|---|
| Catechin | 10⁻⁸ | 102.89±10.21 (48.42) | 101.93±2.27 (52.79) | 1.09 |
| | 10⁻⁷ | 106.72±12.84 (50.22) | 102.57±1.04 (53.12) | 1.06 |
| | 10⁻⁶ | 108.85±8.87 (51.22) | 103.71±9.07 (53.71) | 1.05 |
| | 10⁻⁵ | 128.00±8.74** (60.23) | 115.59±13.39* (59.86) | 1.28 |
| (-)Epicatec hin-3-O-gal late((-)-Epi catechin gallate) | 10⁻⁹ | 121.95±10.76 (57.32) | 97.75±17.66 (53.82) | 0.94 |
| | 10⁻⁸ | 170.00±13.87** (79.90) | 131.16±15.95* (77.72) | 0.97 |
| | 10⁻⁷ | 200.50±13.72** (94.24) | 159.55±28.00** (87.84) | 0.93 |
| | 10⁻⁶ | 192.40±27.72** (90.43) | 148.13±4.22** (81.56) | 0.90 |
| | 10⁻⁵ | 171.88±22.57** (80.79) | 137.80±15.93** (75.87) | 0.94 |
| Naringenin-4'-O-glucos ide (Naringenin -4'-*0-β-D*-gl ucopyranos ide) | 10⁻⁹ | 110.04±5.59 (53.76) | 137.90±9.40** (69.45) | 1.29 |
| | 10⁻⁸ | 157.62±14.54** (77.00) | 180.98±10.46** (91.14) | 1.18 |
| | *10⁻⁷* | 182.34±5.97** (89.08) | 200.66±5.22** (101.06) | 1.13 |
| | 10⁻⁶ | 198.72±3.99** (97.08) | 207.11±15.76** (104.30) | 1.07 |
| | 10⁻⁵ | 188.07±18.61** (91.88) | 215.85±22.32** (108.71) | 1.18 |
| Phlorizin (Phlorizin) | 10⁻⁸ | 105.43±13.57 (55.41) | 109.38±8.72 (59.27) | 1.07 |
| | 10⁻⁷ | 118.40±4.20 (62.23) | 116.14±8.86 (62.93) | 1.01 |
| | 10⁻⁶ | 122.09±11.33* (64.17) | 133.79±5.45** (72.50) | 1.13 |
| | 10⁻⁵ | 137.71±8.44** (72.38) | 147.95±10.29** (80.17) | 1.11 |

| | | | | |
|---|---|---|---|---|
| In the table, *P<0.05, **P<0.01, in comparison with the control | | | | |

**Table 3: Effects of the anthraquinone compounds on ERα or ERβ transcription activity in Hela cells**

| Sample | Concentration (M) | ERα (% of the control) mean±S.D. (% of estradiol) | ERβ (% of the control) mean±S.D. (% of estradiol) | ERβ/ERα (both % of estradiol at 10⁻⁸M) |
|---|---|---|---|---|
| Chrysophanol (Chrysophanol) | 10⁻⁹ | 84.53±14.21 (35.43) | 86.99±23.16 (44.25) | 1.25 |
| | 10⁻⁸ | 80.15±13.88 (33.59) | 76.85±8.82 (39.09) | 1.16 |
| | 10⁻⁷ | 97.77±8.47 (40.98) | 83.12±4.78 (42.28) | 1.03 |
| | 10⁻⁶ | 122.89±8.94** (51.51) | 120.43±12.98** (61.26) | 1.19 |
| Emodin (Emodin) | 10⁻⁹ | 101.27±15.90 (44.27) | 104.68±13.96 (47.31) | 1.07 |
| | 10⁻⁸ | 108.48±27.45 (47.43) | 104.92±9.08 (47.42) | 1.00 |
| | 10⁻⁷ | 109.96±11.33 (48.07) | 106.60±10.76 (48.18) | 1.00 |
| | 10⁻⁶ | 146.40±18.07** (64.01) | 145.21±8.06** (65.63) | 1.03 |

| | | | | |
|---|---|---|---|---|
| In the table, *P<0.05, **P<0.01, in comparison with the control | | | | |

Estrogens mainly exert their effects through estrogen receptor (ER). The studies confirm that ER widely exists in the cardiovascular system, osteoblasts, osteoclasts, mammary gland, and reproductive system, estrogens therefore have wide therapeutical effects, while phytoestrogen is a natural selective estrogen receptor regulating agent, which has similar structure and function with estrogens, may bind to estrogen receptor and has also estrogen like biological activity. The study results show that the compounds of the present invention may activate the estrogen receptor in a concentration dependent manner. Our studies also confirm that many ingredients in *Herba Cynomorii* may bind to ER, increase the transcription activity thereof, thereby confirming they also has estrogen like functions. It also suggests that the catechin, epicatechingallate, naringeninglucoside, phlorizin, emodin, chrysophanol in *Herba Cynomorii* may be used for treatment of climacteric syndrome of women, osteoporosis, degenerative osteoporosis, cardiovascular and cerebrovascular diseases.

## Claims

1. An extract from *Herba Cynomorii songarici,* comprising catechin, (-)epicatechin-3-*O*-gallate, naringenin-4'-O-glucoside and phlorizin, and optionally chrysophanol and/or emodin, which extract is obtained by extraction of *Herba Cynomorii songarici* using water, an alcohol or a water-alcohol mixture, and sequentially petroleum ether, chloroform, and ethyl acetate as the solvent, and is the ethyl acetate extract, the ethyl acetate extract combined with the petroleum ether extract, the ethyl acetate extract combined with the chloroform extract or the ethyl acetate extract combined with the petroleum ether extract and the chloroform extract.

2. The extract of claim 1, wherein the extract is the ethyl acetate extract and comprises catechin, (-) epicatechin-3-O-gallate, naringenin-4'-O-glucoside and phlorizin.

3. The extract of claim 1, wherein the extract is the ethyl acetate extract combined with the petroleum ether extract and comprises catechin, (-) epicatechin-3-O-gallate, naringenin-4'-*O*-glucoside, phlorizin and chrysophanol.

4. The extract of claim 1, wherein the extract is the ethyl acetate extract combined with the chloroform extract and comprises catechin, (-) epicatechin-3-O-gallate, naringenin-4'-*O*-glucoside, phlorizin and emodin.

5. The extract of claim 1, wherein the extract is the mixture of the ethyl acetate extract, the petroleum extract and the chloroform extract and comprises catechin, (-) epicatechin-3-O-gallate, naringenin-4'-*O*-glucoside, phlorizin, chrysophanol and emodin.

6. The extract from *Herba Cynomorii songarici* according to claim 1, which is prepared by a process comprising the following steps: extracting *Herba Cynomorii songarici* with an aqueous ethanol solution; extracting the obtained alcohol extract with petroleum ether, and concentrating the ether layer to obtain a petroleum ether portion; extracting the residue of petroleum ether extraction with chloroform, and concentrating the chloroform layer to obtain a chloroform portion; extracting the residue of chloroform extraction with ethyl acetate, and concentrating the ethyl acetate layer to obtain an ethyl acetate portion; and combining the above ethyl acetate portion with no portion, with the petroleum ether portion, with the chloroform portion, or with the petroleum ether portion and the chloroform portion to obtain the extract.

7. The extract of claim 6, wherein the extract is the ethyl acetate extract and comprises catechin, (-) epicatechin-3-O-gallate, naringenin-4'-O-glucoside and phlorizin.

8. The extract of claim 6, wherein the extract is the ethyl acetate extract combined with the petroleum ether extract and comprises catechin, (-) epicatechin-3-O-gallate, naringenin-4'-*O*-glucoside, phlorizin and chrysophanol.

9. The extract of claim 6, wherein the extract is the ethyl acetate extract combined with the chloroform extract and comprises catechin, (-) epicatechin-3-O-gallate, naringenin-4'-*O*-glucoside, phlorizin and emodin.

10. The extract of claim 6, wherein the extract is the mixture of the ethyl acetate extract, the petroleum extract and the chloroform extract and comprises catechin, (-) epicatechin-3-O-gallate, naringenin-4'-*O*-glucoside, phlorizin, chrysophanol and emodin.

11. The extract of any one of claims 1 to 10 wherein the total amount of ingredients selected from the group consisting of: chrysophanol, emodin, catechin, (-) epicatechin-3-O-gallate, naringenin-4'-*O*-glucoside, and phlorizin is 2-20% (w/w) compared to the total amount of the extraction.

12. The extract from *Herba Cynomorii songarici* according to any one of claims 1 to 11 for use in the treatment and/or prophylaxis of a disease selected from the group consisting of: climacteric syndrome, osteoporosis, degenerative osteoporosis, cardiovascular and cerebrovascular diseases, and breast cancer.

13. Process for preparing an extract from *Herba Cynomorii songarici,* said extract comprising catechin, (-)epicatechin-3-*O*-gallate, naringenin-4'-O-glucoside and phlorizin, and optionally chrysophanol and/or emodin, comprising the following steps: extracting *Herba Cynomorii songarici* with an aqueous ethanol solution; extracting the obtained alcohol extract with petroleum ether, and concentrating the ether layer to obtain a petroleum ether portion; extracting the residue of petroleum ether extraction with chloroform, and concentrating the chloroform layer to obtain a chloroform portion; extracting the residue of chloroform extraction with ethyl acetate, and concentrating the ethyl acetate layer to obtain an ethyl acetate portion; and combining the above ethyl acetate portion with no portion, with the petroleum ether portion ,with the chloroform portion, or with the petroleum ether portion and the chloroform portion to obtain the extract.

## Patentansprüche

1. Extrakt aus *Herba Cynomorii songarici,* umfassend Catechin, (-)-Epi-catechin-3-O-gallat, Naringenin-4'-O-glucosid und Phlorizin, und optional Chrysophanol und/oder Emodin, welcher erhalten wird durch Extraktion von *Herba Cynomorii songarici* unter Verwendung von Wasser, einem Alkohol oder einem Wasser-Alkohol-Gemisch, und nacheinander Petrolether, Chloroform und Ethylacetat als Lösungsmittel, und welcher der Ethylacetatextrakt, der Ethylacetatextrakt kombiniert mit dem Petroletherextrakt, der Ethylacetatextrakt kombiniert mit dem Chloroformextrakt oder der Ethylacetatextrakt kombiniert mit dem Petroletherextrakt und dem Chloroformextrakt ist.

2. Extrakt gemäß Anspruch 1, wobei der Extrakt der Ethylacetatextrakt ist und Catechin, (-)-Epicatechin-3-O-gallat, Naringenin-4'-O-glucosid und Phlorizin umfasst.

3. Extrakt gemäß Anspruch 1, wobei der Extrakt der Ethylacetatextrakt kombiniert mit dem Petroletherextrakt ist und Catechin, (-)-Epicatechin-3-O-gallat, Naringenin-4'-O-glucosid, Phlorizin und Chrysophanol umfasst.

4. Extrakt gemäß Anspruch 1, wobei der Extrakt der Ethylacetatextrakt kombiniert mit dem Chloroformextrakt ist und Catechin, (-)-Epicatechin-3-O-gallat, Naringenin-4'-O-glucosid, Phlorizin und Emodin umfasst.

5. Extrakt gemäß Anspruch 1, wobei der Extrakt das Gemisch aus dem Ethylacetatextrakt, dem Petroletherextrakt und dem Chloroformextrakt ist und Catechin, (-)-Epicatechin-3-O-gallat, Naringenin-4'-O-glucosid, Phlorizin, Chrysophanol und Emodin umfasst.

6. Extrakt aus *Herba Cynomorii songarici* gemäß Anspruch 1, welcher hergestellt wird durch ein Verfahren, das die folgenden Schritte umfasst:
Extrahieren von *Herba Cynomorii songarici* mit einer wässrigen Ethanollösung; Extrahieren des erhaltenen Alkoholextrakts mit Petrolether, und Konzentrieren der Etherschicht, um eine Petroletherfraktion zu erhalten; Extrahieren des Rückstands der Petroletherextraktion mit Chloroform, und Konzentrieren der Chloroformschicht, um eine Chloroformfraktion zu erhalten; Extrahieren des Rückstands der Chloroformextraktion mit Ethylacetat, und Konzentrieren der Ethylacetatschicht, um eine Ethylacetatfraktion zu erhalten; und
Kombinieren der vorstehenden Ethylacetatfraktion mit keiner Fraktion, mit der Petroletherfraktion, mit der Chloroformfraktion, oder mit der Petroletherfraktion und der Chloroformfraktion, um den Extrakt zu erhalten.

7. Extrakt gemäß Anspruch 6, wobei der Extrakt der Ethylacetatextrakt ist und Catechin, (-)-Epicatechin-3-O-gallat, Naringenin-4'-O-glucosid und Phlorizin umfasst.

8. Extrakt gemäß Anspruch 6, wobei der Extrakt der Ethylacetatextrakt kombiniert mit dem Petroletherextrakt ist und Catechin, (-)-Epicatechin-3-O-gallat, Naringenin-4'-O-glucosid, Phlorizin und Chrysophanol umfasst.

9. Extrakt gemäß Anspruch 6, wobei der Extrakt der Ethylacetatextrakt kombiniert mit dem Chloroformextrakt ist und Catechin, (-)-Epicatechin-3-O-gallat, Naringenin-4'-O-glucosid, Phlorizin und Emodin umfasst.

10. Extrakt gemäß Anspruch 6, wobei der Extrakt das Gemisch aus dem Ethylacetatextrakt, dem Petroletherextrakt und dem Chloroformextrakt ist und Catechin, (-)-Epicatechin-3-O-gallat, Naringenin-4'-O-glucosid, Phlorizin, Chrysophanol und Emodin umfasst.

11. Extrakt gemäß einem der Ansprüche 1 bis 10, wobei die Gesamtmenge an Inhaltsstoffen ausgewählt aus der Gruppe bestehend aus Chrysophanol, Emodin, Catechin, (-)-Epicatechin-3-O-gallat, Naringenin-4'-O-glucosid und Phlorizin 2 - 20 Gewichts-% verglichen mit der Gesamtmenge der Extraktion beträgt.

12. Extrakt aus *Herba Cynomorii songarici* gemäß einem der Ansprüche 1 bis 11 zur Verwendung in der Behandlung und/oder Prophylaxe einer Krankheit, ausgewählt aus der Gruppe bestehend aus klimakterischem Syndrom, Osteoporose, degenerativer Osteoporose, kardiovaskulären und zerebrovaskulären Krankheiten und Brustkrebs.

13. Verfahren zur Herstellung eines Extrakts aus *Herba Cynomorii songarici,* wobei der Extrakt Catechin, (-)-Epicatechin-3-O-gallat, Naringenin-4'-O-glucosid und Phlorizin und optional Chrysophanol und/oder Emodin umfasst, umfassend die folgenden Schritte: Extrahieren von *Herba Cynomorii songarici* mit einer wässrigen Ethanollösung; Extrahieren des erhaltenen Alkoholextrakts mit Petrolether, und Konzentrieren der Etherschicht, um eine Petroletherfraktion zu erhalten; Extrahieren des Rückstands der Petroletherextraktion mit Chloroform, und Konzentrieren der Chloroformschicht, um eine Chloroformfraktion zu erhalten; Extrahieren des Rückstands der Chloroformextraktion mit Ethylacetat, und Konzentrieren der Ethylacetatschicht, um eine Ethylacetatfraktion zu erhalten; und Kombinieren der vorstehenden Ethylacetatfraktion mit keiner Fraktion, mit der Petroletherfraktion, mit der Chloroformfraktion, oder mit der Petroletherfraktion und der Chloroformfraktion, um den Extrakt zu erhalten.

## Revendications

1. Extrait *d'Herba Cynomorii songarici,* comprenant de la catéchine, du 3-0-gallate de (-)épicatéchine, du naringénine-4'-O-glucoside et de la phlorizine ainsi que, facultativement, du chrysophanol et/ou de l'émodine, extrait qui est obtenu en soumettant à une extraction l'*Herba Cynomorii songarici* avec de l'eau, un alcool ou un mélange eau-alcool, et successivement de l'éther de pétrole, du chloroforme et de l'acétate d'éthyle comme solvant, et qui est l'extrait à l'acétate d'éthyle, l'extrait à l'acétate d'éthyle combiné avec l'extrait à l'éther de pétrole, l'extrait à l'acétate d'éthyle combiné avec l'extrait au chloroforme ou bien l'extrait à l'acétate d'éthyle combiné avec l'extrait à l'éther de pétrole et l'extrait au chloroforme.

2. Extrait selon la revendication 1, l'extrait étant l'extrait à l'acétate d'éthyle et comprenant de la catéchine, du 3-0-gallate de (-)épicatéchine, du naringénine-4'-0-glucoside et de la phlorizine.

3. Extrait selon la revendication 1, l'extrait étant l'extrait à l'acétate d'éthyle combiné avec l'extrait à l'éther de pétrole et comprenant de la catéchine, du 3-0-gallate de (-)épicatéchine, du naringénine-4'-O-glucoside, de la phlorizine et du chrysophanol.

4. Extrait selon la revendication 1, l'extrait étant l'extrait à l'acétate d'éthyle combiné avec l'extrait au chloroforme et comprenant de la catéchine, du 3-0-gallate de (-)épicatéchine, du naringénine-4'-O-glucoside, de la phlorizine et de l'émodine.

5. Extrait selon la revendication 1, l'extrait étant le mélange de l'extrait à l'acétate d'éthyle, de l'extrait au pétrole et de l'extrait au chloroforme et comprenant de la catéchine, du 3-0-gallate de (-)épicatéchine, du naringénine-4'-O-glucoside, de la phlorizine, du chrysophanol et de l'émodine.

6. Extrait *d'Herba Cynomorii songarici* selon la revendication 1, qui est préparé par un procédé comprenant les étapes suivantes : extraction *d'Herba Cynomorii songarici* avec une solution aqueuse d'éthanol ; extraction de l'extrait alcoolique obtenu avec de l'éther de pétrole, et concentration de la phase éthérée pour obtenir une portion à l'éther de pétrole ; extraction du résidu de l'extraction à l'éther de pétrole avec du chloroforme, et concentration de la phase chloroformique pour obtenir une portion chloroformique ; extraction du résidu de l'extraction au chloroforme avec de l'acétate d'éthyle, et concentration de la phase d'acétate d'éthyle pour obtenir une portion à l'acétate d'éthyle ; et combinaison de la portion à l'acétate d'éthyle précitée avec aucune portion, avec la portion à l'éther de pétrole, avec la portion chloroformique, ou bien avec la portion à l'éther de pétrole et la portion chloroformique pour obtenir l'extrait.

7. Extrait selon la revendication 6, l'extrait étant l'extrait à l'acétate d'éthyle et comprenant de la catéchine, du 3-O-gallate de (-)épicatéchine, du naringénine-4'-O-glucoside et de la phlorizine.

8. Extrait selon la revendication 6, l'extrait étant l'extrait à l'acétate d'éthyle combiné avec l'extrait à l'éther de pétrole et comprenant de la catéchine, du 3-0-gallate de (-)épicatéchine, du naringénine-4'-O-glucoside, de la phlorizine et du chrysophanol.

9. Extrait selon la revendication 6, l'extrait étant l'extrait à l'acétate d'éthyle combiné avec l'extrait au chloroforme et comprenant de la catéchine, du 3-0-gallate de (-)épicatéchine, du naringénine-4'-O-glucoside, de la phlorizine et de l'émodine.

10. Extrait selon la revendication 6, l'extrait étant le mélange de l'extrait à l'acétate d'éthyle, de l'extrait au pétrole et de l'extrait au chloroforme et comprenant de la catéchine, du 3-O-gallate de (-)épicatéchine, du naringénine-4'-O-glucoside, de la phlorizine, du chrysophanol et de l'émodine.

11. Extrait selon l'une quelconque des revendications 1 à 10, dans lequel la quantité totale d'ingrédients choisis dans le groupe consistant en : chrysophanol, émodine, catéchine, 3-O-gallate de (-)épicatéchine, naringénine-4'-O-glucoside et phlorizine va de 2 à 20 % (en poids/poids) comparativement à la quantité totale de l'extraction.

12. Extrait *d'Herba Cynomorii songarici* selon l'une quelconque des revendications 1 à 11, pour une utilisation dans le traitement et/ou la prophylaxie d'une maladie choisie dans le groupe consistant en : le syndrome climatère, l'ostéoporose, l'ostéoporose dégénérative, des maladies cardiovasculaires et vasculaires cérébrales et le cancer du sein.

13. Procédé pour préparer un extrait *d'Herba Cynomorii songarici,* ledit extrait comprenant de la catéchine, du 3-O-gallate de (-)épicatéchine, du naringénine-4'-O-glucoside et de la phlorizine ainsi que, facultativement, du chrysophanol et/ou de l'émodine, comprenant les étapes suivantes : extraction *d'Herba Cynomorii songarici* avec une solution aqueuse d'éthanol ; extraction de l'extrait alcoolique obtenu avec de l'éther de pétrole, et concentration de la phase éthérée pour obtenir une portion à l'éther de pétrole ; extraction du résidu de l'extraction à l'éther de pétrole avec du chloroforme, et concentration de la phase chloroformique pour obtenir une portion chloroformique ; extraction du résidu de l'extraction au chloroforme avec de l'acétate d'éthyle, et concentration de la phase d'acétate d'éthyle pour obtenir une portion à l'acétate d'éthyle ; et combinaison de la portion à l'acétate d'éthyle précitée avec aucune portion, avec la portion à l'éther de pétrole, avec la portion chloroformique, ou bien avec la portion à l'éther de pétrole et la portion chloroformique pour obtenir l'extrait.
